# EUROPÄISCHE PATENTSCHRIFT

(11) **EP 0 873 315 B1**
(45) Veröffentlichungstag und Bekanntmachung des Hinweises auf die Patenterteilung: **23.04.2003**
(21) Anmeldenummer: 96945501.3
(22) Anmeldetag: 11.10.1996
(51) Int. Cl.: C07D 221/18, A61K 31/44, A01N 43/42

(54) **PHENANTHRIDINDERIVATE, VERFAHREN ZU IHRER HERSTELLUNG UND ARZNEIMITTEL, ENTHALTEND PHENANTHRIDINDERIVATE**
PHENANTHRIDINE DERIVATIVES, METHODS OF PRODUCING THEM AND MEDICAMENTS CONTAINING PHENANTHRIDINE DERIVATIVES
DERIVES DE PHENANTHRIDINE, PROCEDES PERMETTANT DE LES PREPARER ET MEDICAMENTS CONTENANT DES PHENANTHRIDINES

(30) Priorität: 13.10.1995 DE 19538088
(43) Veröffentlichungstag der Anmeldung: 28.10.1998
(73) Patentinhaber: Clement, Bernd, D-24106 Kiel (DE)
(72) Erfinder: CLEMENT, Bernd, D-24106 Kiel (DE); WEIDE, Matthias, D-24220 Flintbek (DE)
(74) Vertreter: Pfenning, Meinig & Partner
(86) Internationale Anmeldenummer: DE9601958
(87) Internationale Veröffentlichungsnummer: WO97014683

(56) Entgegenhaltungen:
- EP-A- 0 487 930
- J.MED.CHEM., Bd. 36, Nr. 23, 1993, Seiten 3686-92, XP000652079 JANIN ET AL: "Synthesis and evaluation of New 6-Amino-Substituted Benzo[c]phenanthridine Derivatives" in der Anmeldung erwähnt
- TETRAHEDRON, Bd. 49, Nr. 45, 1993, Seiten 10305-316, XP002028822 JANIN ET AL: "A Formal New Access to the Benzo[c]phenanthridine Alkaloids, Synthesis of Nitidine and O-Methyl Fagorine Analogues" in der Anmeldung erwähnt
- PATENT ABSTRACTS OF JAPAN vol. 14, no. 562 (C-788), 13.Dezember 1990 & JP 02 243629 A (NIPPON KAYAKU CO. LTD.), 27.September 1990,

## Beschreibung

Die Erfindung betrifft neue Phenanthridinderivate, die in 6-Stellung eine Aminogruppe aufweisen, ein Verfahren zu deren Herstellung sowie Arzneimittel, enthaltend diese Phenanthridinderivate.

Bisher bekannte Synthesen von Benzo[c]phenanthridinen, ihren 11,12-Dihydroderivaten und ähnlichen Verbindungen sind sehr komplex. Hier sind die Methoden von Robinson et al. über die Bischler-Napieralski-Zyklisierung sowie von Ninomiya et al. durch Photozyklisierung von Enamiden oder von Shamma et al. und Cushman et al. über die Dickmann-Thorpe-Zyklisierung zu nennen, die alle über sehr viele Reaktionsschritte verlaufen (siehe I. Ninomya und T. Naito: Synthesis of the benzo[c]phenanthridine alkaloids. Recent. Dev. Nat. Carbon compd. 10, 11-90 (1984) und dort zitierte Literatur).

Weiterhin sind aus Pharmazie 44, S. 593-597 (1989), Benzo[c]phenanthridinderivate und ihre antitumorale Wirkung bekannt. Weitere Phenanthridinderivate sind in Tetrahedron 49, S. 10305-10316 (1993), und in J. Chem. Soc. Perkin Trans I, S. 1137-1140 (1983) und in J. Me. Chem. 36, S. 3686-3692 (1993) beschrieben. Bei den Publikationen in J. Med. Chem. und Tetrahedron werden zwar auch derivate mit einer Aminogruppe in 6-Stellung beschrieben, die aber in jedem Fall substituiert ist. Andere Derivate sind bisher nicht bekanntgeworden. Dies ist vor allem darauf zurückzuführen, daß die bisher bekanntgewordenen Derivate auf Syntheseverfahren beruhten, die aufwendig und komplex sind. Eine Herstellung von anderen Phenanthridinderivaten war deshalb bisher nicht möglich.

Ausgehend hiervon ist es die Aufgabe der vorliegenden Erfindung, neue Phenanthridinderivate, ein Verfahren zu ihrer Herstellung sowie deren Anwendung anzugeben.

Die Aufgabe wird in bezug auf die Phenanthridinderivate durch die kennzeichnenden Merkmale des Anspruches 1, in bezug auf das Herstellungsverfahren durch die Merkmale des Anspruches 5 und in bezug auf das Arzneimittel durch Anspruch 10 gelöst. In den Ansprüchen 12 und 13 wird die erfindungsgemäße Verwendung der Phenanthridinderivate beschrieben. Die Unteransprüche zeigen vorteilhafte Weiterbildungen auf.

Gemäß Anspruch 1 sind die neuen Phenanthridinderivate durch die allgemeinen Formeln I und II 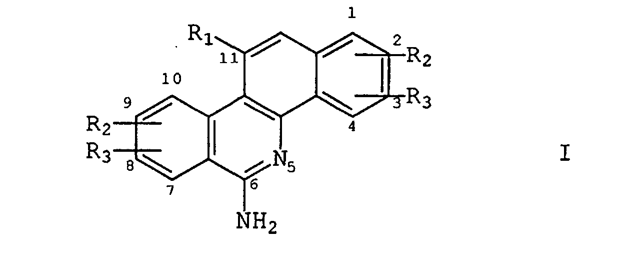 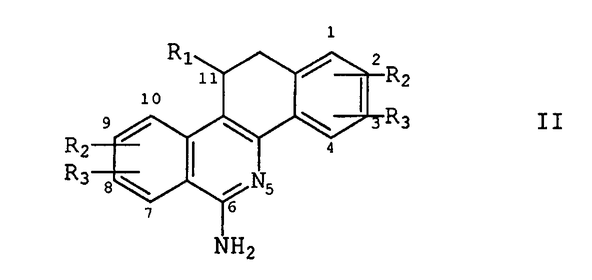 definiert, worin R₁ und R₂ und R₃, die gleich oder verschieden sein können, ein Wasserstoffatom, einen Alkyloxyrest, einen Alkylenoxyrest, ein Halogenatom oder eine Nitrogruppe bedeuten.

Unter einem aromatischen carbocyclischen Rest R₁ sind insbesondere Benzol, Naphthalin, Anthracen, Phenanthren oder Pyren, oder Furan, Thiophen, Pyridin, 1,2,4-Oxdiazol, 1,2,3-Triazol, Benzofuran, Benzoxazol, Benzimidazol, Benzthiazol sowie den entsprechenden Naphtho-Analogen der genannten Benzo-Fünfringheterocyclen, weiterhin Indol, Chinolin oder Isochinolin zu verstehen. Die aromatischen carbocyclischen oder heterocyclischen Reste können einfach oder mehrfach substituiert sein.

Die Substituenten sind ausgewählt aus der Gruppe Monoaminogruppen, Alkylaminogruppen, Dialkylaminogruppen, Alkylgruppen, Alkoxygruppen, Alkylenoxygruppen und Halogene, d.h. unter den Reaktionsbedingungen inerte Gruppen und/oder Atome.

Aufgrund der aufgefundenen pharmakologischen Eigenschaften sind insbesondere diejenigen Derivate von ein unsubstituierter Phenylrest, ein Phenylrest mit einer oder mehreren Methoxygruppen oder eine *N,N*-Dimethylaminofunktion ist. Hervorzuheben sind hierbei diejenigen Derivate, bei denen R₁ ein substituierter oder unsubstituierter Phenylrest, insbesondere 2,4-Dimethoxyphenyl oder 3,4-Dimethoxyphenyl ist. Besonders bevorzugt ist dabei das 2,4-Dimethoxyphenylderivat.

Die erfindungsgemäßen Phenanthridinderivate bilden ohne weiteres physiologisch annehmbare Salze. Solche Salze sind z.B. Salze mit anorganischen und organischen Säuren, z.B. Dihydrochloride, Hydrobromide und Sulfate. Besonders gut geeignete Salze von organischen Säuren werden mit aliphatischen Mono- und Dicarbonsäuren gebildet. Beispiele für solche Salze sind Acetate, Maleate und Fumarate.

Die Verbindungen konnten durch IR- und HNMR-Analyse bestätigt werden.

Die Erfindung betrifft weiterhin ein Verfahren zur Herstellung von Phenanthridinderivaten. Die Anmelderin konnte überraschenderweise zeigen, daß es möglich ist, die erfindungsgemäßen Phenanthridinderivate durch einfache Umsetzung von entsprechend substituierten Aldehyden mit entsprechend substituierten Methylbenzonitril zu erhalten. Im einzelnen wird dabei so vorgegangen, daß eine Umsetzung von einem Aldehyd der Formel III

R₁-CHO III

worin R₁ die vorgenannte Bedeutung besitzt, mit 2 Mol eines 2-Methylbenzonitrils der Formel IV 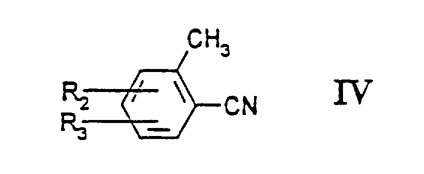 worin R₂ und R₃ die obengenannte Bedeutung besitzen, in Gegenwart von Base in einem aprotischen dipolaren Lösungsmittel durchgeführt wird und nach Isolierung in einem weiteren Schritt nach allgemein gültigen Methoden mit einem geeigneten Dehydrierungsmittel in Ab- oder Anwesenheit von Lösungsmittel eine Dehydrierung erfolgt. Der Reaktionsablauf läßt sich wie folgt darstellen:

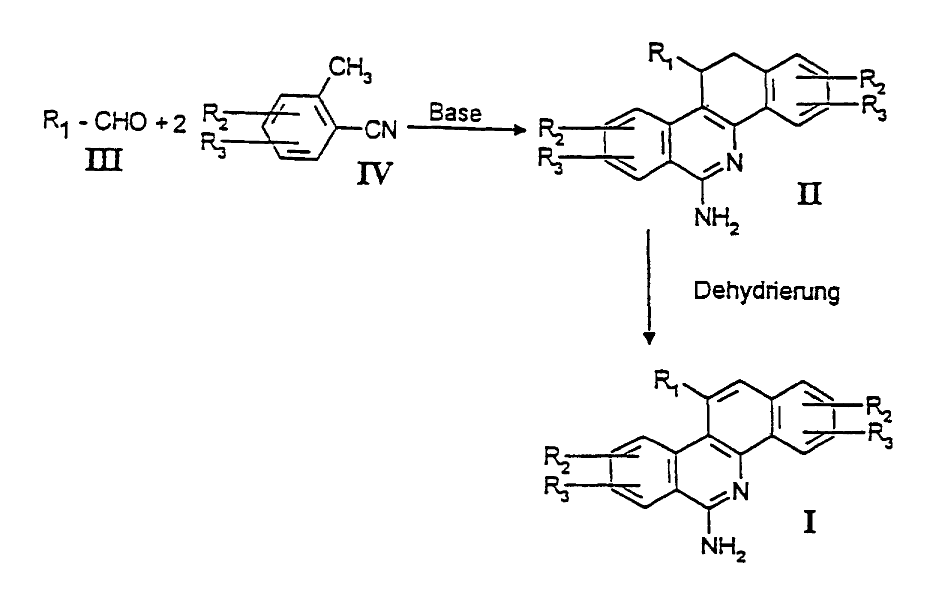

Als aprotische dipolare Lösungsmittel für die erfindungsgemäße Reaktion sind vorzugsweise Amide wie Dimethylformamid, Dimethylacetamid, Diäthylacetamid, Hexamethyl-phosphorsäure-trisamid und Hexamethyl-phosphorsäure-trisamid oder Harnstoffe wie Tetramethylharnstoff, 1,3-Dimethyltetrahydro-2-pyrimidinon und 1,3-Dimethylimidazolidinon oder Dimethylsulfoxid verwendbar.

Als Base können beispielsweise eingesetzt werden:
Alkali- oder Erdalkalihydride wie Natriumhydrid, Alkaliamide wie Natriumamid, Natriummethylacetamid, Alkali-, Erdalkali-, oder Aluminiumalkoholate wie Kalium-*tert.*-butylat, Natriummethylat, Natriumäthylat oder Aluminiumäthylat.

Die Reaktion kann wie folgt durchgeführt werden: Zu einer Lösung von Base in einem geeigneten dipolaren aprotischen Lösungsmittel wird unter Inertbegasung eine Lösung der Verbindung III und IV in demselben Lösungsmittel langsam zugetropft. Nach mehrstündigem Rühren bei 35 °C bis 50 °C unter Inertbegasung wird der Ansatz auf Eiswasser gegossen und mit einem geeigneten organischen Lösungsmittel ausgeschüttelt. Die organische Phase wird eingeengt und aus dem Rückstand durch Einleiten einer Halogenwasserstoffsäure oder Schütteln mit einer geeigneten anorganischen oder organischen Säure das 6-Amino-11,12-dihydrobenzo[c]phenanthidin II gefällt oder bei Verwendung einer wäßrigen Säurelösung aus der wäßrigen Phase nach Neutralisation und Ausfallen der Base isoliert. Das 6-Amino-11,12-dihydrobenzo[c]phenanthridin II kann dann nach allgemein gültigen Methoden mit einem geeigneten Dehydrierungsmittel in Gegenwart oder Abwesenheit eines inerten Lösungsmittels zum 6-Aminobenzo[c]phenanthridin I dehydriert werden.

Besonders hervorzuheben beim erfindungsgemäßen Verfahren ist, daß sich hiermit Phenanthridinderivate synthetisieren lassen, die in 11-Stellung einen substituierten oder unsubstituierten Phenylrest aufweisen. Es ist überraschend, daß die Synthese durch die vorstehend beschriebene einfache Reaktion möglich ist, wobei aufgrund der einzusetzenden Ausgangssubstanzen eine große Variationsbreite in bezug auf die zu erhaltenden Edukte besteht.

Es wurde nun gefunden, daß die vorstehend beschriebenen Phenanthridinderivate ausgezeichnete antitumorale, antimikrobielle, antifungizide, antivirale und antiinflammatorische Eigenschaften besitzen. Zur Untersuchung der pharmakologischen Eigenschaften wurden die Verbindungen der allgemeinen Formel I und II in einem "*in-vitro*-Antitumor-Screening" des National Cancer Institute (NCI), Bethesdal, Maryland, USA, untersucht. Getestet wurden gegen 58 verschiedene humanpathogene Tumorzellreihen, die neun Krebsarten entstammten (Leukämie, nichtkleinzelliges Lugencarcinom, Dickdarmkrebs, ZNS-Krebs, Melanom, Eierstockkrebs, Nierenkrebs, Prostatakrebs, Brustkrebs). Zur Ermittlung der Wirksamkeit wurden die Turmozellen den Verbindungen über zwei Tage ausgesetzt und danach indirekt über die Bestimmung der Proteinbiomasse mit Sulforhodamin B (SRB) die Wachstumshemmung ermittelt. Unbehandelte Kulturen dienten als Referenz.

Bei diesen Untersuchungen zeigte z.B. 6-Amino-11-(2,4-dimethoxyphenyl)benzo[c]phenanthridiniumperchlorat Wachstumshemmungen. Überraschenderweise wies die Verbindung Aktivitäten auf, die außerhalb der Kategorie von in ähnlicher Weise studierten Antitumorverbindungen liegt, so daß hier ein ganz neues Wirkungsspektrum erzielt wird.

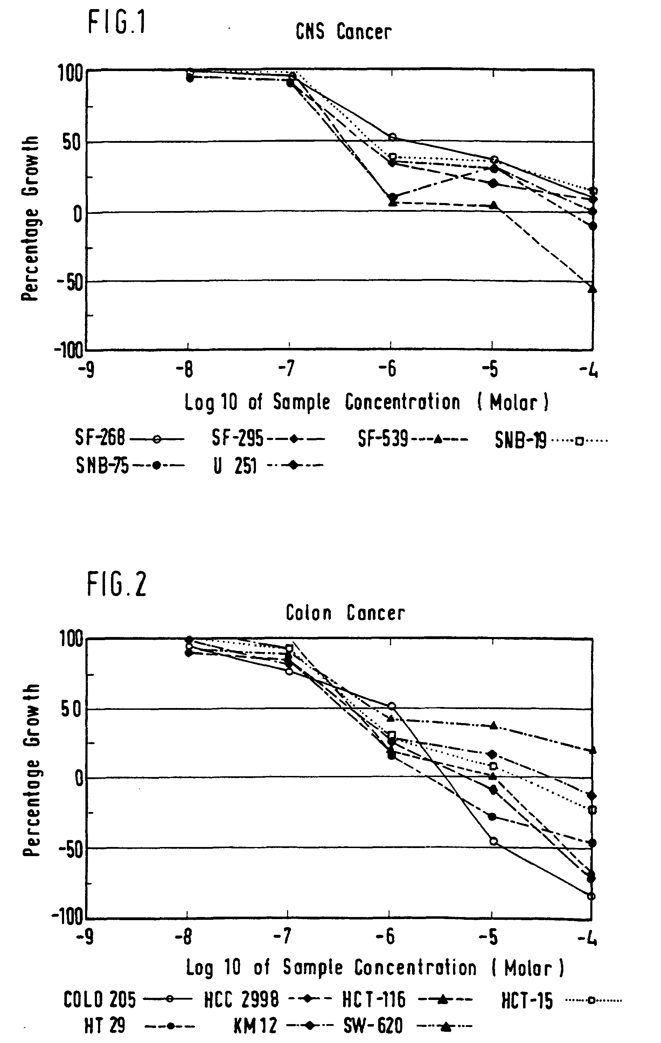

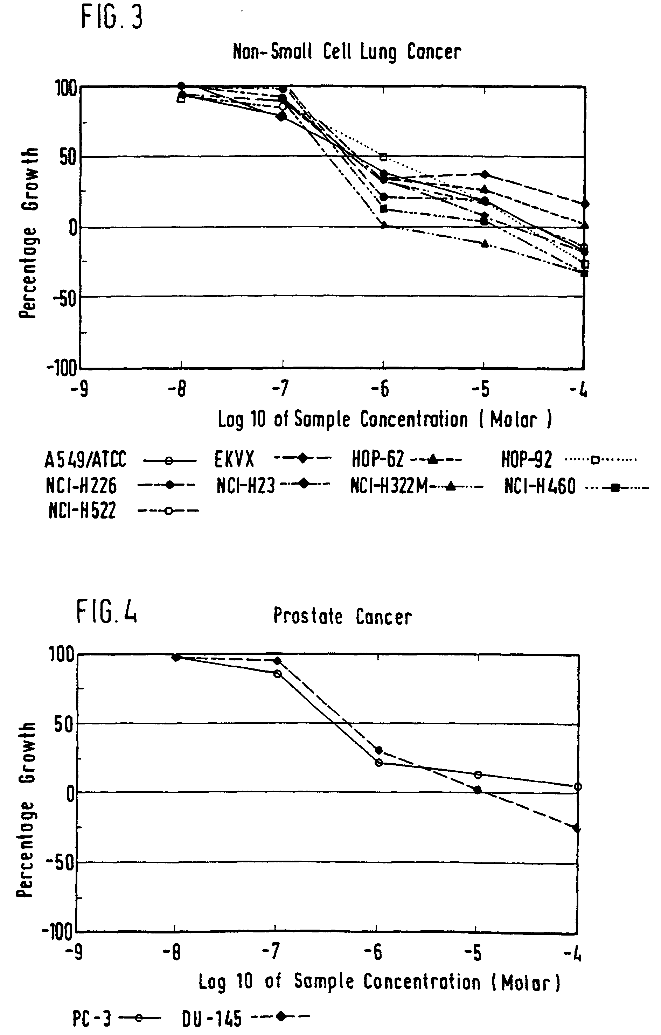

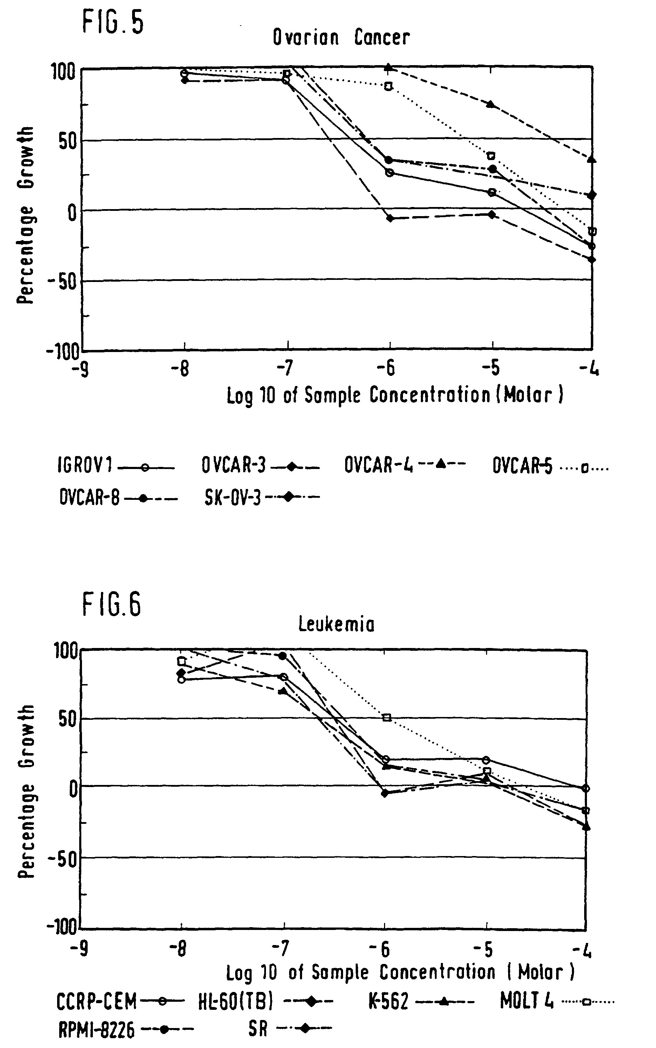

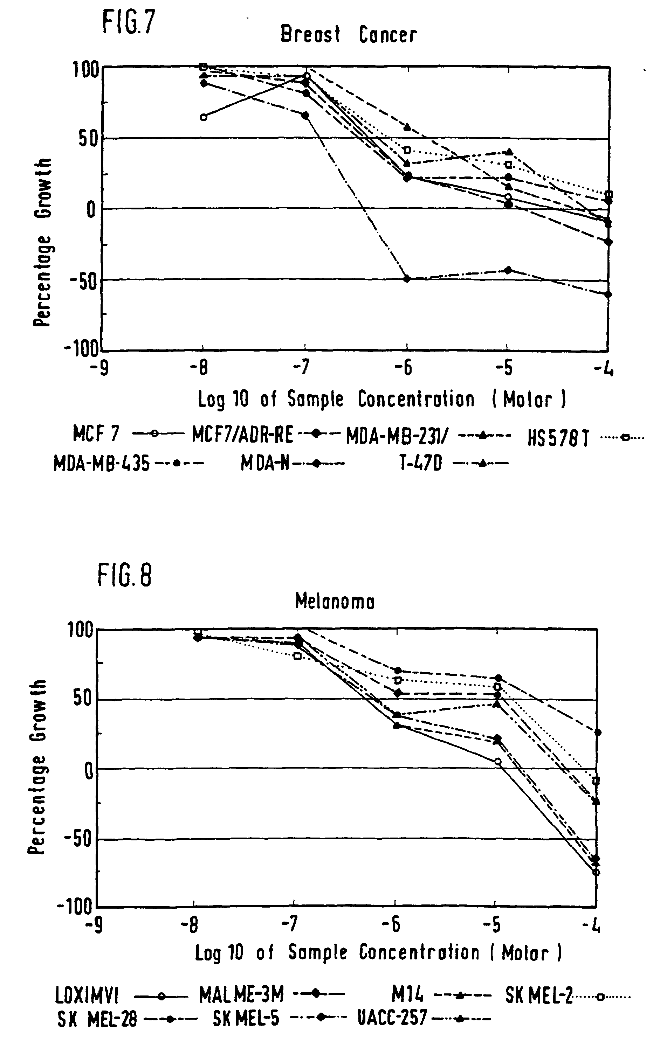

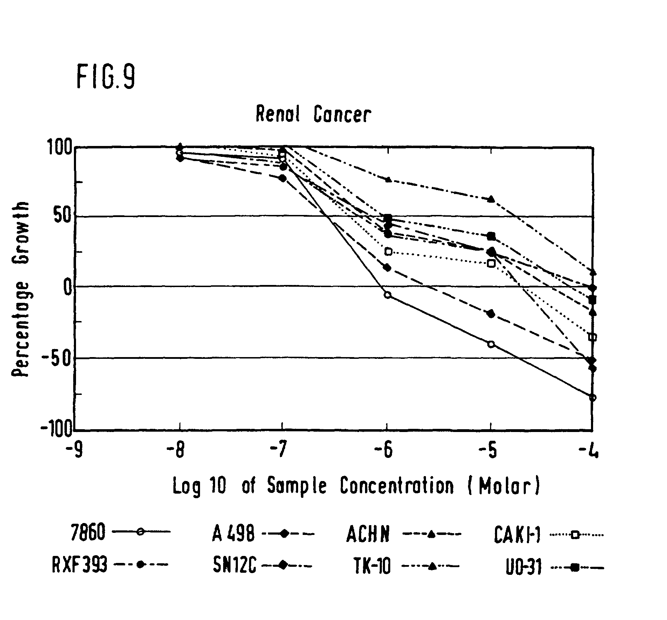

Von den vorliegenden Daten sind beispielhaft für diese Verbindung Dosis-Wirkungs-Kurven in den Figuren 1 bis 9 wiedergegeben. Die neun verschiedenen Figuren beinhalten die verschiedenen Krebsformen. Aufgetragen ist jeweils das prozentuale Wachstum in Abhängigkeit von der Konzentration der Verbindung (als log₁₀ der molaren Konzentrationen). Die einzelnen Kurven einer jeden Krebsart sind verschiedene Zellinien dieser Krebsform, die als Legenden in ihren üblichen Abkürzungen erscheinen. Horizontale Linien in den Abbildungen bedeuten Prozentwachstum +100, +50, 0, -50 und -100. 100 % Wachstum bedeutet z.B. keinen Unterschied zum Wachstum nach zwei Tagen ohne Substanzzusatz. Man erkennt bei den einzelnen Kurven, daß sich mit steigenden Konzentrationen der Substanz das Prozentwachstum verkleinert.

Die Erfindung betrifft deshalb auch Arzneimittel, die die vorstehend beschriebenen Phenanthridinderivate enthalten. Das Arzneimittel enthält dabei mindestens ein Phenanthridinderivat in der vorstehend beschriebenen Weise zusammen mit mindestens einem inerten pharmazeutisch annehmbaren Träger oder Verdünnungsmittel. Bevorzugt ist als Phenanthridinderivat ein Derivat der allgemeinen Formel I enthalten, bei dem R₁ ein 2,4-Methoxyphenylrest ist, und R₂ und R₃ Wasserstoff. Die erfindungsgemäße Verbindung kann oral, topisch oder parenteral oder in Form von Subpositoren verabreicht werden. Der bevorzugte Verabreichungsweg ist die perorale Verabreichung. Sie kann in Form der Base oder als physiologisch annehmbares Salz verabreicht werden. Sie wird im allgemeinen mit einem pharmazeutisch annehmbaren Träger oder Verdünnungsmittel vermengt, um ein Arzneimittel zu ergeben. Für die orale Verabreichung kann das Arzneimittel am zweckmäßigsten in Form von Kapseln oder Tabletten vorliegen, die auch Tabletten mit verzögerter Freisetzung sein können. Die Arzneimittel können auch in Form von Dragees oder in Sirupform vorliegen. Geeignete topische Zubereitungen sind z.B. Salzen, Lotionen, Cremes, Pulver und Sprays.

Die Erfindung wird nachfolgend anhand mehrerer Ausführungsbeispiele näher erläutert.

### Ausführungsbeispiele:

### Herstellung der 6-Amino-11,12-dihydrobenzo[c]phenanthridine II:

### Beispiel 1:

### 6 Amino-11,12-dihydro-11-phenylbenzo[c]phenanthridiniumchlorid

Zu einer Lösung von 2,47 mg (22 mmol) KOBu^{t} in 20 ml DMPU wird unter Stickstoffbegasung eine Lösung von 1,06 g (10 mmol) Benzaldehyd und 2.34 g (20 mmol), 2-Methylbenzonitril in 5 ml DMPU langsam zugetropft. Nach fünf Stunden Rühren bei 35 °C unter Stickstoffrbegasung wird der Ansatz auf eine Lösung von 2.2 g (40 mmol) Ammoniumchlorid in 100 ml Eiswasser gegossen und dreimal mit 100 ml Dichlormethan ausgeschüttelt. Die organische Phase wird über Watte filtriert und auf etwa 100 ml einrotiert und mit 3 N Salzsäure kräftig geschüttelt. Der entstandene Niederschlag wird abgesaugt, mit Dichlormethan gewaschen und getrocknet. Nach Umkristallisieren aus Methanol/Dichlormethan erhält man 6-Amino-11,12-dihydro-11-phenylbenzo[c]-phenanthridiniumchlorid. Hellgelbe Plättchen, Ausb.: 52 % d. Th., Schmp. 355 °C. - IR (KBr): v = 3446 cm⁻¹, 3076, 1662, 1620, 1570. - ¹H NMR (400 MHz, [D₆]DMSO): δ = 3.18 (mc, 1H, 12-H), 3.56 (mc, 1H, 12-H), 4,95 (mc, 1H, 11-H), 7.09 (mc, 5H, C6H5-), 7.24 (d, 1H, Ar-H), 7.35 (t, 1H, Ar-H), 7.44 (t, 1H, Ar-H), 7.74 (mc, 1H, Ar-H), 7.91 (mc, 2H, Ar-H), 8.3 (d, 1H, Ar-H), 8.61 (d, 1H, Ar-H), 9.3 (br, 2H, -NH₂), 13.7 (br, 1H, ≡N⁺-H).

| | | | | |
|---|---|---|---|---|
| C₂₃H₁₉N₂Cl(358.87) | Ber. | C 76.98 | H 5.34 | N 7.81 |
| | Gef. | C 76.52 | H 5.37 | N 7.75 |

### Beispiel 2:

### 6-Amino-11,12-dihydro-11-(3,4-dimethoxyphenyl)benzo-[c]phenanthridiniumchlorid

Analog Beispiel 2. Hellgelbe Nadeln, Aus.: 53 % d. Th., Schmp. 205 °C (Methanol/Wasser). - IR (KBr): v = 3438 cm⁻¹, 3268, 3106, 2938, 1648, 1616, 1584. - ¹H NMR (400 MHz, [D₆]DMSO): δ = 3.08 (mc, 1H, 12-H), 3.42 (mc, 1H, 12-H), 3.61 (s, 3H, -OCH₃), 3.99 (s, 3H, -OCH₃), 5.02 (mc, 1H, 11-H), 6.04 (mc, 1H, Ar-H), 6.21 (mc, 1H, Ar-H), 6.61 (mc, 1H, Ar-H), 7.20 (d, 1H, Ar-H), 7.34 (t, 1H, Ar-H), 7.43 (t, 1H, Ar-H), 7.63 (d, 1H, Ar-H), 7.73 (t, 1H, Ar-H), 7.91 (t, 1H, Ar-H), 8.36 (d, 1H, Ar-H), 8.61 (d, 1H, Ar-H), 9.56 (br, 2H, -NH₂), 13.85 (br, 1H, ≡N⁺-H).

| | | | | |
|---|---|---|---|---|
| C₂₅H₂₃N₂O₂C1(418.92) | Ber. | C 71.68 | H 5.53 | N 6.69 |
| | Gef. | C 70.95 | H 5.37 | N 6.80 |

### Herstellung der 6-Aminobenzo[c]phenanthridine I:

### Beispiel 1:

### 6-Aminobenzo[c]phenanthridiniumperchlorat

Zu einer Lösung von 250 mg (1.02 mmol) 6-Amino-11,12-dihydrobenzo[c]phenanthridin in 15 ml Dioxan wird eine Lösung von 404 mg (1.7 mmol) DDQ in 35 ml Dioxan gegeben und vier Stunden unter Rückfluß erhitzt. Die erkaltete Lösung wird anschließend auf eine Natriumhydrogencarbonatlösung gegossen und mit Diethylether ausgeschüttelt. Die Diethyletherphase wird einmal mit verdünnter Natriumhydrogencarbonatlösung und dreimal mit Wasser gewaschen. Nach Zugabe von 70 % Perchlorsäure wird ein Niederschlag erhalten. Nach Trocknen und Umkristallisieren aus Methanol braune Nadeln, Ausb.; 44 % d. Th., Schmp. 325 °C. - IR (KBr): ν = 3404 cm⁻¹, 3348, 3298, 3276, 3234, 1666, 1616.- ¹H NMR (300 MHz, [D₆]DMSO): δ = 7.82 (mc, 3H, Ar-H), 8.0 (d, 1H, Ar-H), 8.13 (mc, 2H, Ar-H), 8.56 (mc, 2H, Ar-H), 8.69 (d, 1H, Ar-H), 8.83 (d, 1H, Ar-H), 9.73 (br, 2H, -NH₂), 12.84 (br, 1H, ≡N⁺-H).

| | | | | |
|---|---|---|---|---|
| C₁₇H₁₃N₂O₄Cl(344,06) | Ber. | C 59.29 | H 3.81 | N 8.14 |
| | Gef. | C 59.23 | H 3.83 | N 8.24 |

### Beispiel 2:

### 6-Amino-11-phenylbenzo[c]phenanthridiniumperchlorat

Anlog Beispiel 1. Graubraune Nadeln, Ausb.: 50 % d. Th., Schmp. 345 °C. - IR (KBr) ν = 3412 cm⁻¹, 3358, 3310, 3226, 1668, 1642, 1612. - ¹H NMR (300 MHz, [D₆]DMSO): δ = 7.51 (mc, 7H, Ar-H), 7.80 (mc, 4H, Ar-H), 8.15 (d, 1H, Ar-H), 8.66 (mc, 2H, Ar-H), 9.88 (br, 2H, -NH₂), 12.8 (br, 1H, ≡N⁺-H).

| | | | | |
|---|---|---|---|---|
| C₂₃H₁₇N₂O₄Cl(420.09) | Ber. | C 65.70 | H 4.08 | N 6.67 |
| | Gef. | C 65.67 | H 4.03 | N 6.67 |

### Beispiel 3:

### 6-Amino-11-(2,4-dimethoxyphenyl)benzo[c]phenanthridiniumperchlorat

Analog Beispiel 1. Dunkelbraune Nadeln, Ausb.: 45 % d. Th., Schmp. 336 °C. - IR (KBr): ν = 3418 cm⁻¹, 3352, 3302, 3270, 1660, 1608. - ¹H NMR (300 MHz, [D₆]DMSO): δ = 3.38 (s, 3H, -OCH₃), 3.87 (s, 3H, -OCH₃), 6.69 (mc, 1H, Ar-H), 6.77 (mc, 1H, Ar-H), 7.34 (mc, 1H, Ar-H), 7.77 (mc, 6H, Ar-H), 8.11 (mc, 1H, Ar-H), 8.77 (mc, 2H, Ar-H), 9.72 (br, 2H, -NH₂); 12.58 (br, 1H, ≡N⁺-H).

| | | | | |
|---|---|---|---|---|
| C₂₅H₂₁N₂O₆C1(480.19) | Ber. | C 62.49 | H 4.41 | N 5.83 |
| | Gef. | C 62.56 | H 4.30 | N 5.87 |

## Patentansprüche

1. Phenanthridinderivate der allgemeinen Formel I 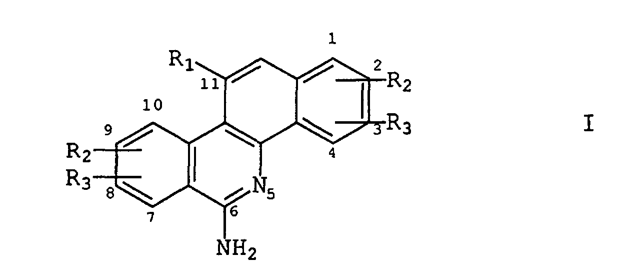 und II 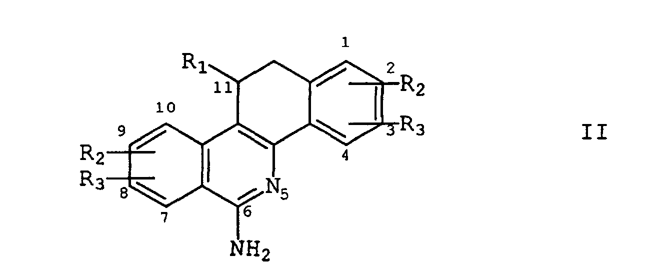 sowie deren Salze, worin R₁ Benzol, Naphthalin, Anthracen, Phenanthren, Pyren, oder Furan, Thiophen, Pyridin, 1,2,4-Oxidazol, 1,2,3-Triazol, Benzofuran, Benzoxazol, Benzimidazol, Benzthiazol sowie ein entsprechendes Naphtho-Analogen der genannten Benzo-Fünfringheterocyclen oder Indol, Chinolin oder Isochinolin ist, wobei R₁ einfach oder mehrfach substituiert sein kann und die Substituenten ausgewählt sind aus der Gruppe Monoamino, Alkylamino, Dialkylamino, Alkyl, Alkoxy, Alkylenoxy und Halogen und R₂ und R₃, die gleich oder verschieden sein können, ein Wasserstoffatom, einen Alkyloxyrest, einen Alkylenoxyrest, ein Halogenatom oder eine Nitrogruppe bedeuten.

2. Phenanthridinderivate nach Anspruch 1,
**dadurch gekennzeichnet, daß** R₁ ein unsubstituierter Phenylrest, oder ein Phenylrest mit einer oder mehreren Methoxygruppen oder ein Rest mit einer *N,N*-Dimethylaminofunktion ist, wobei R₂ und R₃ gleich H sind.

3. Phenanthridinderivat nach Anspruch 2,
**dadurch gekennzeichnet, daß** R₁ ein Phenylrest, ein 2,4- oder 3,4-Methoxyphenylrest ist.

4. Phenanthridinderivat nach Anspruch 3,
**dadurch gekennzeichnet, daß** in der allgemeinen Formel I R₁ ein 2,4-Methoxyphenylrest ist.

5. Verfahren zur Herstellung der Phenanthridinderivate der allgemeinen Formel II nach einem der Ansprüche 1 bis 4,
**dadurch gekennzeichnet,**
**daß** man einen Aldehyd der Formel III R₁-CHO mit einem 2-Methylbenzonitril der Formel IV 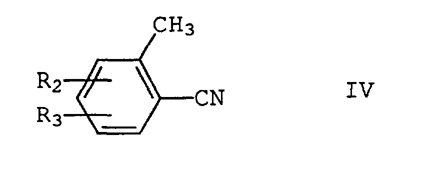 wobei die Reste R₁, R₂ und R₃ die in den Ansprüchen 1 bis 4 angegebenen Bedeutungen besitzen, in Gegenwart von Basen in einem aprotischen dipolaren Lösungsmittel umsetzt.

6. Verfahren zur Herstellung der Phenanthridinderivate der allgemeinen Formel I nach einem der Ansprüche 1 bis 4,
**dadurch gekennzeichnet, daß** man einen Aldehyd der Formel III R₁ CHO mit einem 2-Methylbenzonitril der Formel IV 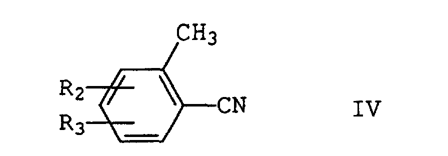 wobei die Reste R₁, R₂ und R₃ die in den Ansprüchen 1 bis 4 angegebenen Bedeutungen besitzen, in Gegenwart von Basen in einem aprotischen dipolaren Lösungsmittel umsetzt, und **daß** dann in einem weiteren Schritt mit einem geeigneten Dehydrierungsmittel in Ab- oder Anwesenheit von Lösungsmitteln eine Dehydrierung erfolgt.

7. Verfahren zur Herstellung der Phenanthridinderivate nach Anspruch 5 oder 6,
**dadurch gekennzeichnet, daß** ein Aldehyd der allgemeinen Formel III eingesetzt wird, bei dem R₁ ein Phenylrest, ein 2,4- oder 3,4-Methoxyphenylrest oder Wasserstoff ist.

8. Verfahren nach Anspruch 7,
**dadurch gekennzeichnet, daß** R₁ ein 2,4- oder 3,4-Methoxyphenylrest ist.

9. Verfahren nach mindestens einem der Ansprüche 5 bis 8,
**dadurch gekennzeichnet, daß** ein 2-Methylbenzonitril der allgemeinen Formel IV eingesetzt wird, bei dem R₂ und R₃ Wasserstoff ist.

10. Arzneimittel,
**dadurch gekennzeichnet,**
**daß** es mindestens eines der Phenanthridinderivate der Ansprüche 1 bis 4 zusammen mit mindestens einem inerten pharmazeutischen annehmbaren Träger oder Verdünnungsmittel enthält.

11. Arzneimittel nach Anspruch 10,
**dadurch gekennzeichnet, daß** es ein Phenanthridinderivat der allgemeinen Formel I enthält, bei dem R₁ ein 2,4-Methoxyphenylrest ist, und R₂ und R₃ Wasserstoff.

12. Phenanthridinderivate nach einem der Ansprüche 1 bis 4 zur Verwendung als Arzneimittel.

13. Verwendung der Phenanthridinderivate nach einem der Ansprüche 1 bis 4 zur Herstellung eines Arzneimittels zur Behandlung von tumoralen, mikrobiellen, fungiziden, viralen und/oder inflammatorischen Erkrankungen.

## Claims

1. Phenanthridine derivatives of the general formula I 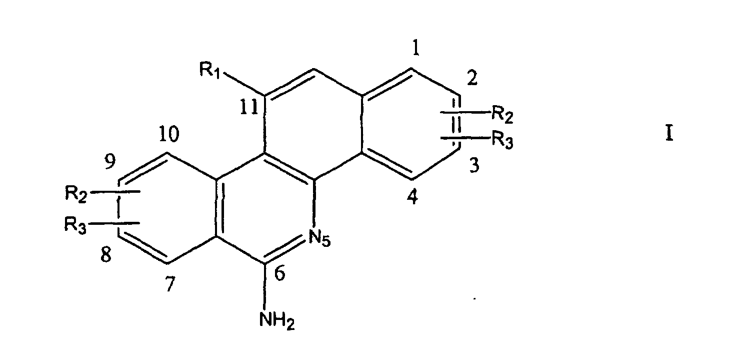 and II 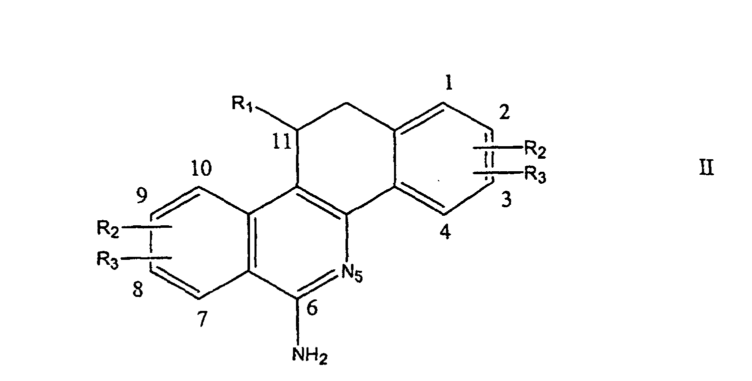 and their salts, where R₁ is benzene, naphthalene, anthracene, phenanthrene, pyrene, or furan, thiophene, pyridine, 1,2,4-oxide azole, 1,2,3-triazole, benzofuran, benzoxazole, benzimidazole, benzothiazole and a corresponding naphtho analogue of the aforementioned benzo five-membered heterocyclic ring, or indole, quinoline or isoquinoline, wherein R₁ can be monosubstituted or polysubstituted and the substituents are selected from the monoamino, alkylamino, dialkylamino, alkyl, alkoxy, alkyleneoxy and halogen group, and R₂ and R₃, which can be the same or different, signify a hydrogen atom, an alkyloxy residue, an alkyleneoxy residue, a halogen atom or a nitro group.

2. Phenanthridine derivatives according to claim 1, **characterised in that** R₁ is an unsubstituted phenyl residue or a phenyl residue with one or more methoxy groups or a residue with an *N,N*-dimethylamino function, wherein R₂ and R₃ are both H.

3. A phenanthridine derivative according to claim 2, **characterised in that** R₁ is a phenyl residue or a 2,4- or 3,4-methoxyphenyl residue.

4. A phenanthridine derivative according to claim 3, **characterised in that**, in the general formula I, R₁ is a 2,4-methoxyphenyl residue.

5. A process for the preparation of the phenanthridine derivatives of the general formula II according to any one claims 1 to 4, **characterised in that** an aldehyde of the formula III R₁-CHO is reacted with a 2-methylbenzonitrile of the formula IV 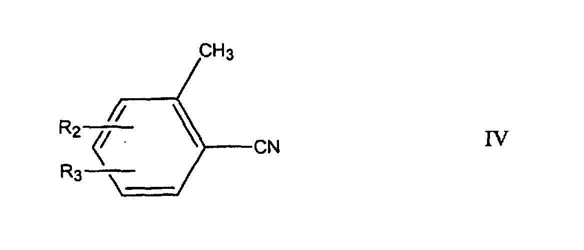 in the presence of bases in an aprotic dipolar solvent, wherein the residues R₁, R₂ and R₃ have the meanings indicated in claims 1 to 4.

6. A process for the preparation of the phenanthridine derivatives of the general formula I according to any one of claims 1 to 4, **characterised in that** an aldehyde of the formula III R₁-CHO is reacted with a 2-methylbenzonitrile of the formula IV 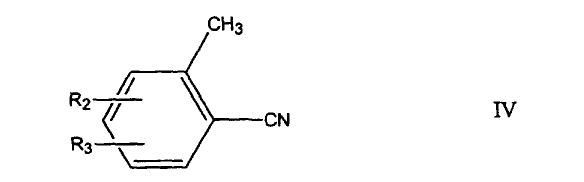 in the presence of bases in an aprotic dipolar solvent, wherein the residues R₁, R₂ and R₃ have the meanings indicated in claims 1 to 4, and **in that** dehydrogenation is then carried out in a further step with a suitable dehydrogenating agent in the absence or presence of solvents.

7. A process for the preparation of the phenanthridine derivatives according to claim 5 or 6, **characterised in that** an aldehyde of the general formula III is used, in which R₁ is a phenyl residue, a 2,4-or 3,4-methoxyphenyl residue or hydrogen.

8. A process according to claim 7, **characterised in that** R₁ is a 2,4-or 3,4-methoxyphenyl residue.

9. A process according to at least one of claims 5 to 8, **characterised in that** a 2-methylbenzonitrile of the general formula IV is used, in which R₂ and R₃ are hydrogen.

10. A medicament, **characterised in that** it contains at least one of the phenanthridine derivatives of claims 1 to 4 together with at least one inert, pharmaceutically acceptable carrier or diluent.

11. A medicament according to claim 10, **characterised in that** it contains a phenanthridine derivative of the general formula I, in which R₁ is a 2,4-methoxyphenyl residue and R₂ and R₃ are hydrogen.

12. Phenanthridine derivatives according to any one of claims 1 to 4 for use as medicaments.

13. Use of the phenanthridine derivatives according to any one of claims 1 to 4 in the preparation of a medicament for the treatment of tumoral, microbial, fungicidal, viral and/or inflammatory diseases.

## Revendications

1. Dérivés de phénanthridine de formules générales I 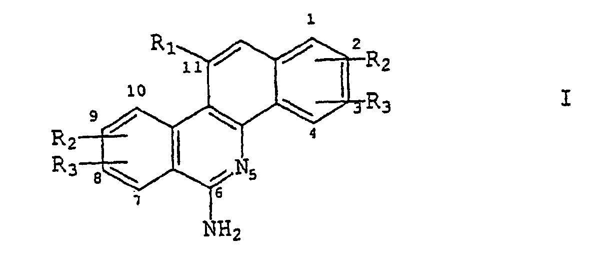 et II 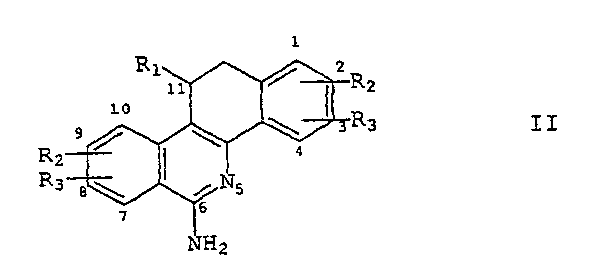 ainsi que leurs sels, formules dans lesquelles R₁ est le benzène, le naphtalène, l'anthracène, le phénanthrène, le pyrène ou le furanne, te thiophène, la pyridine, le 1,2,4-oxidazole, le 1,2,3-triazole, le benzofuranne, le benzoxazole, le benzimidazole, le benzothiazole ainsi qu'un analogue naphto des benzo-hétérocycles pentagonaux cités ou l'indole, la quinoléine ou l'isoquinoléine, R₁ pouvant être une ou plusieurs fois substitué et les substituants étant choisis dans le groupe constitué par des atomes d'halogène et des groupes monoamino, alkylamino, dialkylamino, alkyle, alcoxy et alkylène-oxy, et R₂ et R₃, qui peuvent être identiques ou différents, représentent un atome d'hydrogène ou d'halogène, un radical alkyloxy, un radical alkylène-oxy ou un groupe nitro.

2. Dérivés de phénanthridine selon la revendication 1, **caractérisés en ce que** R₁ est un radical phényle non substitué ou un radical phényle portant un ou plusieurs groupes méthoxy ou un radical ayant une fonction N,N-diméthylamino, R₂ et R₃ représentant H.

3. Dérivé de phénanthridine selon la revendication 2, **caractérisé en ce que** R₁ est un radical phényle ou un radical 2,4- ou 3,4-méthoxyphényle.

4. Dérivé de phénanthridine selon la revendication 3, **caractérisé en ce que**, dans la formule générale I, R₁ est un radical 2,4-méthoxyphényle.

5. Procédé pour la préparation des dérivés de phénanthridine de formule générale II selon l'une quelconque des revendications 1 à 4, **caractérisé en ce qu'**on fait réagir un aldéhyde de formule III R₁-CHO avec un 2-méthylbenzonitrile de formule IV 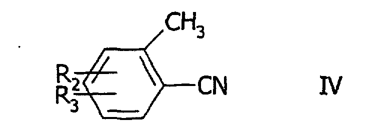 dans laquelle les radicaux R₁, R₂ et R₃ ont les significations données dans les revendications 1 à 4, en présence de bases dans un solvant aprotique dipolaire.

6. Procédé pour la préparation des dérivés de phénanthridine de formule générale I selon l'une quelconque des revendications 1 à 4, **caractérisé en ce qu'**on fait réagir un aldéhyde de formule III R₁-CHO avec un 2-méthylbenzonitrile de formule IV 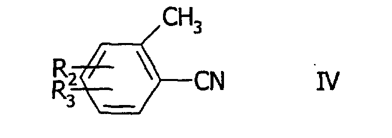 dans laquelle les radicaux R₁, R₂ et R₃ ont les significations données dans les revendications 1 à 4, en présence de bases dans un solvant aprotique dipolaire, et **en ce que**, dans une autre étape, on effectue ensuite une déshydrogénation avec un agent de déshydrogénation approprié, en absence ou en présence de solvants.

7. Procédé pour la préparation des dérivés de phénanthridine selon la revendication 5 ou 6, **caractérisé en ce qu'**on fait réagir un aldéhyde de formule générale III dans lequel R₁ est un radical phényle, un radical 2,4- ou 3,4-méthoxyphényle ou un atome d'hydrogène.

8. Procédé selon la revendication 7, **caractérisé en ce que** R₁ est un radical 2,4- ou 3,4-méthoxyphényle.

9. Procédé selon au moins l'une des revendications 5 à 8, **caractérisé en ce qu'**on utilise un 2-méthylbenzonitrile de formule générale IV, dans lequel R₂ et R₃ représentent un atome d'hydrogène.

10. Médicament **caractérisé en ce qu'**il contient au moins un des dérivés de phénanthridine selon les revendications 1 à 4, conjointement avec au moins un véhicule ou diluant inerte pharmaceutiquement acceptable.

11. Médicament selon la revendication 10, **caractérisé en ce qu'**il contient un dérivé de phénanthridine de formule générale I, dans lequel R₁ est un radical 2,4-méthoxyphényle, et R₂ et R₃ représentent un atome d'hydrogène.

12. Dérivés de phénanthridine selon l'une quelconque des revendications 1 à 4, pour utilisation en tant que médicament.

13. Utilisation des dérivés de phénanthridine selon l'une quelconque des revendications 1 à 4, pour la préparation d'un médicament destiné au traitement de maladies tumorales, microbiennes, fongiques, virales et/ou inflammatoires.
